# EUROPEAN PATENT SPECIFICATION

(11) **EP 1 486 180 B1**
(45) Date of publication and mention of the grant of the patent: **03.12.2008**
(21) Application number: 04013781.2
(22) Date of filing: 11.06.2004
(51) Int. Cl.: A61C 1/18

(54) **Fluid supply connector**
Verbindungsstück zur Flüssigkeitsversorgung
Raccord pour l'approvisionnement de fluides

(30) Priority: 10.06.2003 JP 2003164789
(43) Date of publication of application: 15.12.2004
(73) Proprietor: Nakanishi Inc., Kanuma-shi, Tochigi 322-8666 (JP)
(72) Inventor: Nakanishi, Eiichi, Kanuma-shi Tochigi 322-8666 (JP); Kawakubo, Kiyoshi, Kanuma-shi Tochigi 322-8666 (JP)
(74) Representative: Hauck Patent- und Rechtsanwälte

(56) References cited:
- EP-A- 0 497 576
- DE-A- 3 501 044
- DE-C- 717 669
- US-A- 5 762 495

## Description

The present invention relates to a connector to be connected to fluid passages for supplying at least one of different fluids to a medical handpiece.

Some medical handpieces used for treatment of a site are capable of injecting fluid, such as medicine-containing liquid, water, and air, depending on its usage, from the tip of the handpiece toward the treatment site. An example of such handpieces is a dental handpiece for removing tartar, which has an ultrasonic vibrated-scaler tip at its end, and is required to inject medicine-containing liquid in some cases, or water in some other cases. Wiring and fluid lines extend from the supply sources of electrical power or fluids to the handpiece, and a connector is detachably connected in the middle of such wiring and lines.

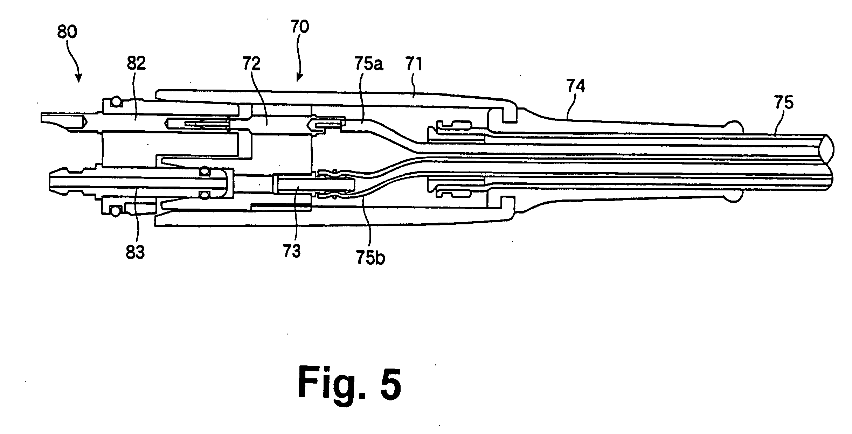

Fig. 5 shows an example of such connectors, which are designated as 70 and 80. The connector 70 is connected to an end of a hose 75 extending toward a handpiece (not shown). The connector 70 has a casing 71 encasing therein a fluid pipe 73 and a plurality of electric terminals 72, to which fluid tube 75b and wiring 75a extending through the hose 75 are connected, respectively. The connector 80 is connected at one end to the rear end of the connector 70, and is connected at the other end to an end of a hose (not shown) extending from supply sources (not shown) of fluid and/or electricity or electrical signals. The connector 80 has a plurality of electric terminals 82 and a fluid pipe 83 inside thereof.

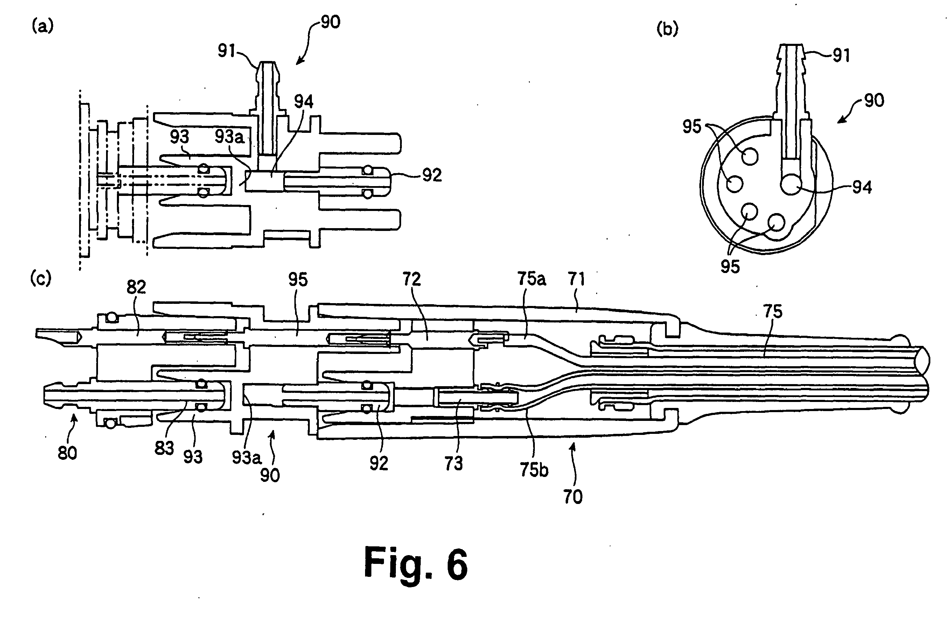

Fig. 6 shows an example of a switching connector 90 for switching connection of tubing from fluid supply sources. This switching connector 90 is located between the connectors 70 and 80. The connector 90 has a plurality of electric terminals 95 therein, and an inlet pipe 91 and an outlet pipe 92 communicating with each other via a conduit 94. A receptor portion 93 into which the fluid pipe 83 of the connector 80 is fit, is formed with a wall 93a for blocking off the stream from the fluid pipe 83 into the conduit 94. With such a structure, the switching connector 90 blocks the fluid supplied to the fluid pipe 83 of the connector 80, and takes another fluid in through the inlet pipe 91 to pass via the outlet pipe 92 into the fluid tube 75b, while the connection between the electric terminals 82 and 72 is maintained by means of the terminals 95.

The above connectors 70 and 80 require the switching connector 90, which is detachably connected between the connectors 70 and 80 depending on the selected fluid to be injected through the tip of the dental handpiece. This attaching/detaching operation is troublesome, and the operation is complicated.

A fluid supply connector for connecting a plurality of fluid lines from fluid supply sources to a medical handpiece according to the introduction portion of patent claim 1 has been disclosed in DE 717 669 C.

It is an object of the present invention to provide a fluid supply connector which is capable of changing the fluid to be injected through the tip of a medical handpiece such as a dental handpiece, without detaching/attaching parts, such as a connector, for each change, without troublesome operations, and in a simple manner.

The fluid supply connector of the invention has been defined in patent claim 1. Further developments and modifications of the invention have been defined in the dependent patent claims.

In patent claim 1, "at least one of said plurality of fluid passages has a check valve provided therein" means that a check valve may be provided in only some of the fluid passages in the fluid supply connector, and is not necessarily provided in the rest of the fluid passages. In this case, a solenoid valve may instead be provided in a fluid supply unit upstream of the fluid passage without a check valve. It should be understood that the recitation of claim 1 includes an embodiment wherein the check valve is provided in all of the fluid passages.

With the above structure, by operating only the predetermined fluid supply source while the others are stopped, the fluid once passed through the check valve is discharged into the fluid passage toward the medical handpiece without flowing into the stopped fluid supply source. Accordingly, different fluids may be injected through the tip of a dental handpiece merely by selectively operating the fluid supply sources.

The present invention will now be explained in detail with reference to the attached drawings showing illustrative examples of the invention, wherein:
Fig. 1 is a schematic view of the overall system wherein a fluid supply connector 10 according to the present invention is applied;

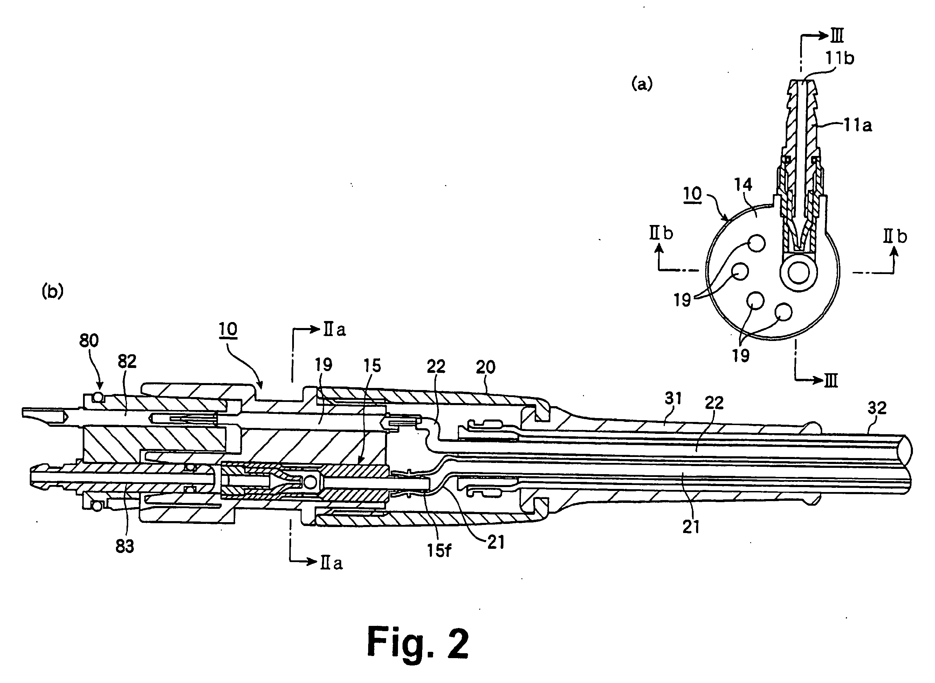

Fig. 2 (a) is a cross-sectional view of a fluid supply connector 10 according to the present invention taken along lines IIa-IIa in Fig. 2(b);
Fig. 2 (b) is a longitudinal sectional view, taken along lines IIb-IIb in Fig. 2 (a), of the fluid supply connector 10 having a hose 32 and a connector 80 attached to the front and rear ends of the connector 10, respectively;
Fig. 3 is a sectional view of the fluid supply connector 10 of Fig. 2, taken along lines III-III in Fig. 2(a);
Fig. 4 is an exploded perspective view of a fluid supply connector according to the present invention;
Fig. 5 is a longitudinal sectional view of prior art connectors; and
Figs. 6(a) to 6(c) are sectional views of prior art connectors.

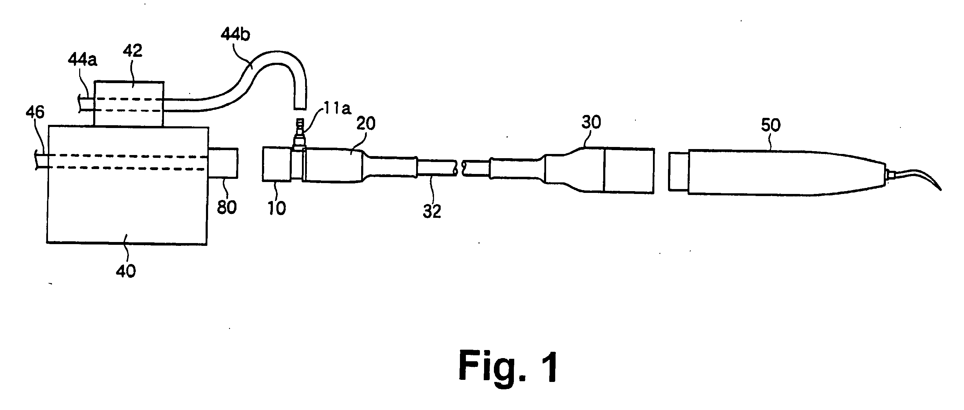

The fluid supply connector 10 according to the present invention may be used in a system shown in Fig. 1, but is not limited to such a system. In the example shown in Fig. 1, the rear end of the connector 10 is connected to a connector 80, which is in turn connected to a supply unit 40 that sends out a first fluid and electricity or electric signals. A tube 46 extends from the supply unit 40 to a supply source of the first fluid. To the front end of the connector 10 is connected a hose 32 extending toward a handpiece 50. The rear end of the hose 32 is fixed to the fluid supply connector 10 by means of a casing 20, and the front end of the hose 32 is fixed to a connector 30 to be connected to the handpiece 50. A tube 44b for supplying a second fluid is connected to the fluid supply connector 10 from the lateral direction, and the other end of the tube 44b is connected via a supply unit 42 to a supply source of the second fluid.

The fluid supply connector 10 is mainly composed of a body 14 including a first fluid passage, a second fluid passage, and a plurality of electric terminals 19 provided therein.

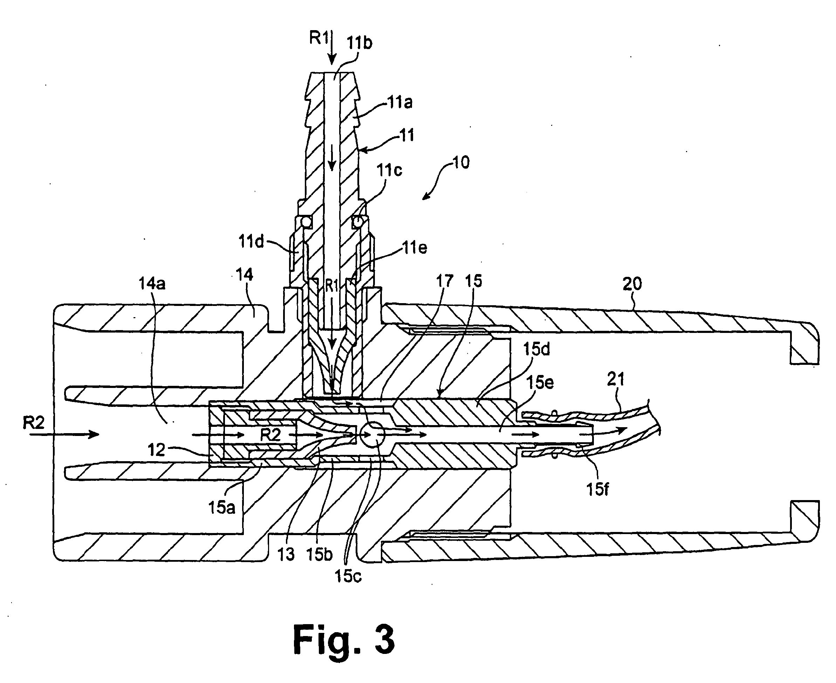

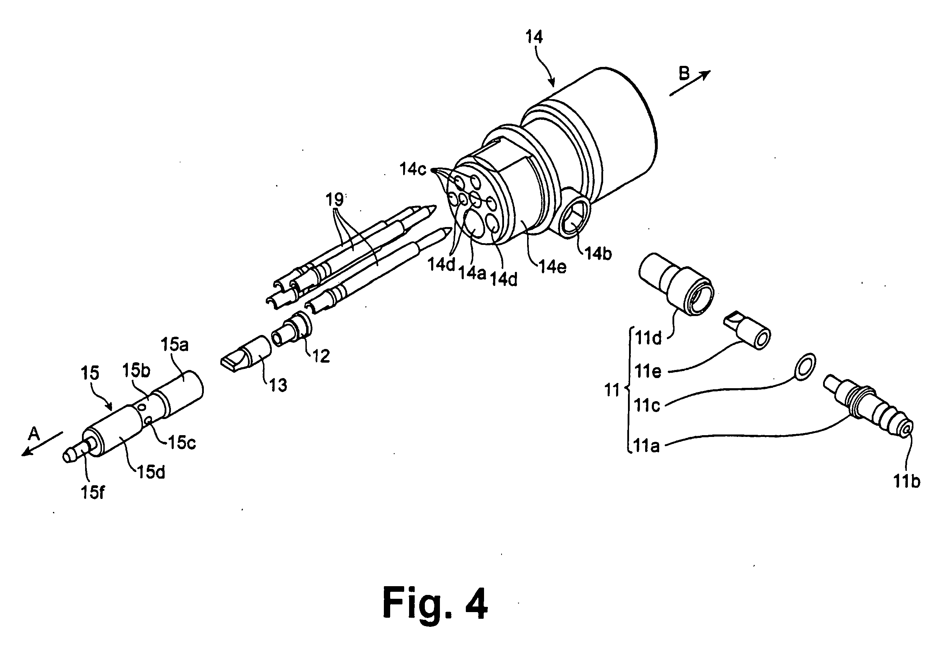

Referring to Figs. 3 and 4, each component is explained. The body 14 has channels 14a and 14c extending axially through the body 14. In each channel 14c, an electric terminal 19 is inserted and seated. A transverse channel 14b is formed in the body 14 in the lateral direction, and is in communication with the axial channel 14a. The body 14 also has a channel 14d left after adjustment of thickness in the molding process of the body 14. Incidentally, in Fig. 4, arrow A shows the direction toward a handpiece, while arrow B shows the direction toward the supply unit 40.

The first fluid passage is formed with a first conduit 15, a check valve 13, and a fixture 12, all inserted and seated in the channel 14a in the body 14. More specifically, the first conduit 15 accommodates the check valve 13 therein, and the fixture 12 is screwed in the rear end of the first conduit 15 for fixing the check valve 13. The first conduit 15, the check valve 13, and the fixture 12 thus integrated are inserted into the channel 14a in the body 14 and seated therein. The first conduit 15 has a main fluid duct 15e extending axially through the conduit 15, a thin extension pipe 15f having a thinned diameter on which a tube 21 is to be attached, and a thinned middle section 15b having a reduced outer diameter. In the thinned middle section 15b, a plurality of apertures 15c are formed to communicate with the internal main duct 15e. The thinned middle section 15b of the first conduit 15 forms an annular gap 17 between the outer surface of the thinned middle section 15b and the inner surface of the channel 14a in the body 14. This gap 17, in cooperation with the apertures 15c, makes the second fluid passage communicate with the main duct 15e of the first conduit 15.

The second fluid passage is formed with an attachment pipe 11d, a check valve 11e, an O-ring 11c, and a tube connector pipe 11a, all inserted and seated in the channel 14b in the body 14. More specifically, the attachment pipe 11d accommodates the check valve 11e therein, and the tube connector pipe 11a is screwed in the rear end of the attachment pipe 11d via the O-ring 11c for fixing the check valve 11e with the front end of the tube connector pipe 11a, so that an integral pipe unit 11 is assembled. This pipe unit 11 is inserted into the channel 14b in the body 14 and seated therein, to thereby form the second fluid passage.

The thus assembled fluid supply connector 10 is connected at one end to the hose 32 extending from the handpiece side. The hose 32 contains a plurality of electric wiring 22 and a fluid tube 21 bundled within a sheath. One end of the hose 32 is fixed to an end of the casing 20 by means of a retainer 31, and the casing 20 is in turn screwed onto one end 14e of the body 14 (Fig. 4). In this state, the fluid tube 21 and the plurality of electric wiring 22 are connected to the thin extension pipe 15f and the plurality of electric terminals 19, respectively, in the casing 20. Incidentally, the connector 80 to be connected to the other end of the fluid supply connector 10 has a plurality of electric terminals 82 and a fluid pipe 83 provided inside thereof.

Next, the operation of the fluid supply connector 10 according to the present invention will now be explained.

In Fig. 1, for example, the tube 46 is connected to a feed pipe of tap water to enable supply of tap water to the first fluid passage of the fluid supply connector 10, whereas the tube 44a is connected to a chemical supply source to enable supply of chemical to the second fluid passage of the fluid supply connector 10. In this state of connection, for supplying only the chemical to the handpiece, the supply of tap water is stopped at the supply unit 40, while the supply unit 42 for the chemical is operated. Then the chemical, as shown by arrow R1 in Fig. 3, enters the tube connector pipe 11a, flows through the check valve 11e into the gap 17, then enters through the apertures 15c into the main duct 15e of the first conduit 15, and flows out through the thin extension pipe 15f into the fluid tube 21 toward the handpiece. Here, since the main duct 15e of the first conduit 15 has the check valve 13 provided therein, the chemical will not flow back into the tap water supply side beyond the check valve 13, but will be sent out only toward the handpiece side.

On the other hand, for supplying only the tap water to the handpiece, the tap water is supplied by means of the supply unit 40 via the tube 46, while the supply of the chemical is stopped at the supply unit 42. Then the tap water, as shown by arrow R2 in Fig. 3, enters through the channel 14a of the body 14 into the first conduit 15, flows through the check valve 13 into the main duct 15e, and flows out through the thin extension pipe 15f into the fluid tube 21 toward the handpiece. Here, the tap water may enter the attachment pipe 11d of the second fluid passage through the apertures 15c of the first conduit 15 and the gap 17, but is prevented by the check valve 11e from flowing back into the chemical supply side, and is sent out only toward the handpiece side.

As discussed above, the fluid supply connector 10 of the present invention facilitates changing of the fluid to be injected through the tip of a dental handpiece, merely by connecting each fluid supply tubes extending from different fluid supply sources to the first and second fluid passages, respectively, and operating one of the fluid supply sources with the other being stopped. Consequently, connectors do not have to be detached/attached depending on the fluid to be supplied, as in the prior art connectors, and thus the troubles in operating the connector are eliminated and the operation is simplified.

In the above embodiment of the fluid supply connector, both the first and second fluid passages are described to have a check valve. However, only one of the first and second fluid passages may be provided with a check valve, while the other may be free of a check valve. In this case, any valve for preventing backflow, such as a solenoid valve, may be provided in the fluid supply unit upstream of the fluid passage having no check valve.

The fluid supply connector according to the present invention is disposed in the fluid supply passages extending from different fluid supply sources, and is capable of changing the fluid to be injected through the tip of a handpiece from one fluid to the other, merely by selectively operating the fluid supply sources. Thus, change of the fluid to be injected may be facilitated, and the operation may be simplified.

## Claims

1. A fluid supply connector (10) for connecting a plurality of fluid lines (44b, 46) from fluid supply sources to a medical handpiece (50), comprising:
a plurality of inlet ends, each to be connected with a fluid line (44b, 46) extending from a fluid supply source,
an outlet end (15f) to be connected with a fluid line (21) extending toward a medical handpiece, and
a plurality of fluid passages (14a, 14b) each connecting one of said inlet ends to said outlet end (15f), wherein at least one of said plurality of fluid passages (14a, 14b) has a check valve (13, 11e) provided therein for preventing flow of fluid into said inlet ends,
**characterized in that** one of said plurality of fluid passages (14a) is formed with a conduit (15) having a thinned middle section (15b) with a reduced outer diameter, said thinned middle section (15b) having apertures (15c) for communicating the interior (15e) of said conduit with another of said plurality of fluid passages (14b).

2. The connector of claim 1, further comprising a terminal (19) for connecting with wiring (82) extending from a supply source of electricity or electrical signals, and with wiring (22) extending toward a medial handpiece.

3. The connector of claim 1 or 2, wherein said connector has first and second fluid passages (14a, 14b), with said first fluid passage (14a) having said check valve (13) provided therein.

4. The connector of claim 3, wherein said second fluid passage (14b) also has said check valve (11e) provided therein.

5. The connector of any of the preceding claims, wherein said conduit (15) is seated in said one of the plurality of fluid passages (14a), and said thinned middle section (15b) forms an annular gap (17) between the outer surface of the thinned middle section (15b) and the inner surface of said one of the plurality of fluid passages (14a), wherein said annular gap (17), in cooperation with said apertures (15c), makes said another of the plurality of fluid passages (14b) communicate with the interior (15e) of said conduit (15).

## Patentansprüche

1. Verbindungsstück zur Fluidversorgung (10) zum Anschließen einer Mehrzahl von Fluidleitungen (44b, 46) von Fluidversorgungsquellen an ein medizinisches Ansatzstück (50), mit:
mehreren Einlassenden, die jeweils mit einer von einer Fluidversorgungsquelle ausgehenden Fluidleitung (44b, 46) zu verbinden sind,
einem Auslassende (15f), das mit einer in Richtung zu einem medizinischen Ansatzstück verlaufenden Fluidleitung (21) zu verbinden ist, und
einer Mehrzahl von Fluidkanälen (14a, 14b), die jeweils eines der Einlassenden mit dem Auslassende (15f) verbinden, wobei in wenigstens einem von den mehreren Fluidkanälen (14a, 14b) ein Regelventil (13, 11e) vorgesehen ist, um einen Fluidstrom in die Einlassenden zu verhindern,
**dadurch gekennzeichnet, dass** der eine von den mehreren Fluidkanälen (14a) mit einem Leitungsrohr (15) mit einem verschwächten Mittelabschnitt (15b) mit einem verkleinerten Außendurchmesser ausgebildet ist, wobei der verschwächte Mittelabschnitt (15b) Öffnungen (15c) zum Verbinden des Innern (15e) des Leitungsrohres mit einem anderen von den mehreren Fluidkanälen (14b) aufweist.

2. Verbindungsstück nach Anspruch 1, außerdem mit einem Anschlussteil (19) zum Anschließen von Drähten (82), die von einer Versorgungsquelle für Elektrizität oder elektrische Signale ausgehen, und Drähten (22), die zu einem medizinischen Ansatzstück (50) hin verlaufen.

3. Verbindungsstück nach Anspruch 1 oder 2, wobei das Verbindungsstück einen ersten und einen zweiten Fluidkanal (14a, 14b) aufweist und in dem ersten Fluidkanal (14a) das Rückschlagventil (13) vorgesehen ist.

4. Verbindungsstück nach Anspruch 3, wobei in dem zweiten Fluidkanal (14b) ebenfalls das Rückschlagventil (11e) vorgesehen ist.

5. Verbindungsstück nach einem der vorhergehenden Ansprüche, wobei das Leitungsrohr (15) in dem einen von den mehreren Fluidkanälen (14a) aufgenommen ist und der verschwächte Mittelabschnitt (15b) einen Ringspalt (17) zwischen der Außenfläche des verschwächten Mittelabschnitts (15b) und der Innenfläche des einen von den mehreren Fluidkanälen (14a) bildet, wobei der Ringspalt (17) im Zusammenwirken mit den Öffnungen (15c) den anderen von der Mehrzahl von Fluidkanälen (14b) mit dem Innern (15e) des Leitungsrohres (15) in Verbindung setzt.

## Revendications

1. Raccord d'alimentation en fluide (10) pour raccorder une pluralité de lignes de fluide (44b, 46) allant de sources d'alimentation en fluide à un porte-outil médical (50), comprenant :
une pluralité d'extrémités d'entrée, chacune à raccorder à une ligne de fluide (44b, 46) s'étendant à partir d'une source d'alimentation en fluide,
une extrémité de sortie (15f) à raccorder à une ligne de fluide (21) s'étendant vers un porte-outil médical, et
une pluralité de passages de fluide (14a, 14b) raccordant chacun l'une desdites extrémités d'entrée à ladite extrémité de sortie (15f), dans lequel au moins l'un de ladite pluralité de passages de fluide (14a, 14b) comporte une vanne d'arrêt (13, 11e) prévue dans celui-ci pour empêcher un écoulement de fluide dans lesdites extrémités d'entrée,
**caractérisé en ce que** l'un de ladite pluralité de passages de fluide (14a) est formé avec une conduite (15) ayant une section centrale amincie (15b) d'un diamètre extérieur réduit, ladite section centrale amincie (15b) ayant des ouvertures (15c) pour faire communiquer l'intérieur (15e) de ladite conduite avec un autre de ladite pluralité de passages de fluide (14b).

2. Raccord selon la revendication 1, comprenant en outre une borne (19) pour se connecter à un câblage (82) s'étendant d'une source d'alimentation d'électricité ou de signaux électriques, et le câblage (22) s'étendant vers un porte-outil médical.

3. Raccord selon la revendication 1 ou 2, dans lequel le raccord présente des premier et deuxième passages de fluide (14a, 14b), ledit premier passage de fluide (14a) comportant à l'intérieur de celui-ci ladite vanne d'arrêt (13).

4. Raccord selon la revendication 3, dans lequel ledit deuxième passage de fluide (14b) comporte aussi ladite vanne d'arrêt (11e) à l'intérieur de celui-ci.

5. Raccord selon l'une quelconque des revendications précédentes, dans lequel ladite conduite (15) est logée dans ledit un passage de la pluralité de passages de fluide (14a), et ladite section centrale amincie (15b) forme un espace annulaire (17) entre la surface extérieure de la section centrale amincie (15b) et la surface intérieure dudit un passage de la pluralité de passages de fluide (14a), dans lequel ledit espace annulaire (17), en coopération avec lesdites ouvertures (15c), fait communiquer un autre passage de la pluralité de passages de fluide (15b) avec l'intérieur (15e) de ladite conduite (15).
